## Europäisches Patentamt
## European Patent Office
## Office européen des brevets

(19)

(11) Veröffentlichungsnummer: **0 180 737**
**B1**

(12) # EUROPÄISCHE PATENTSCHRIFT

(45) Veröffentlichungstag der Patentschrift:
23.08.89

(51) Int. Cl.⁴: **A 61 K 45/02**, A 61 K 45/06

(21) Anmeldenummer: **85111183.1**

(22) Anmeldetag: **04.09.85**

(54) **Verwendung von Interferon gamma (IFN-gamma) zur Herstellung von Präparationen zur Behandlung rheumatischer Erkrankungen.**

(30) Priorität: **05.10.84 DE 3436638**

(43) Veröffentlichungstag der Anmeldung:
**14.05.86 Patentblatt 86/20**

(45) Bekanntmachung des Hinweises auf die Patenterteilung:
**23.08.89 Patentblatt 89/34**

(84) Benannte Vertragsstaaten:
**AT BE CH DE FR GB IT LI LU NL SE**

(56) Entgegenhaltungen:
**EP-A-0 077 063**
**US-A-4 483 849**

CHEMICAL ABSTRACTS, Band 100, Nr. 21, 21. Mai 1984, Seite 483, Zusammenfassung Nr. 172931d, Columbus, Ohio, US; I.F. BARINSKII et al.: "Successful therapy of autoimmune disease in NZB/W mice with interferon", & VOPR. VIRUSOL. 1984, 29(1), 38-42
CHEMICAL ABSTRACTS, Band 102, Nr. 15, 15. April 1985, Seite 471, Zusammenfassung Nr. 130150j, Columbus, Ohio, US; M.L. STPHENSON et al.: "Immune interferon inhibits collagen synthesis by rheumatoid synovial cells associated with decreased levels of the procollagen mRNAs", & FEBS LETT. 1985, 180(1), 43-50
BIOLOGICAL ABSTRACTS, REPORTS, REVIEWS AND MEETINGS, Band 30, 1985, Zusammenfassung

(73) Patentinhaber: **BIOFERON Biochemische Substanzen GmbH & Co, Erwin- Rentschler-Strasse 21, D-7958 Laupheim 1 (DE)**

(72) Erfinder: **v. Eichborn, Johann- Friedrich, Dr., Humlangen 6, D-7901 Hüttisheim (DE)**
Erfinder: **Link, Franz, Dr., Am Kohlenschacht 5, D-8403 Bad Abbach (DE)**
Erfinder: **Obert, Hans- Joachim, Dr., Adolf- Gröber-Strasse 12, D-7958 Laupheim (DE)**

(74) Vertreter: **Dipl.- Ing. Schwabe, Dr. Dr. Sandmair, Dr. Marx, Stuntzstrasse 16 Postfach 86 02 45, D-8000 München 86 (DE)**

(56) Entgegenhaltungen: (Fortsetzung)
Nr. 13013, Philadelphia, US; G.W. FISHBEIN: "Gamma interferon proves promising in rheumatoid arthritis", & GENET. ENG. LETT. 1985, vol. 5, no. 19, p3

## Beschreibung

Alle bekannten Interferone werden auf Grund von Homologien sowohl zwischen den Nukleotidsequenzen ihrer Strukturgene als auch zwischen ihren Aminosäuresequenzen eindeutig in drei Gruppen eingeteilt, die als IFN-alpha, IFN-beta und IFN-gamma bezeichnet werden.

Die verwendeten Bezeichnungen folgen der neuesten Empfehlung des "Interferon Nomenclature Commitee", die von J. Vilcek 1983 in Archives of Virology, vol. 77, S. 283 - 285, veröffentlicht wurden. Zusätzlich zu den genannten Hauptkriterien kann zur Charakterisierung der IFN-gamma-Gruppe herangezogen werden, daß keine immunologische Kreuzreaktion mit den beiden anderen Gruppen erfolgt sowie ihre biologische Instabilität bei pH 2 im Gegensatz zu IFN-alpha und IFN-beta.

IFN-gamma wird nach Stimulation der Gesamtpopulation der Leukozyten, nach der Art der Präparation üblicherweise als buffy coats bezeichnet, durch Mitogene, Antigene oder spezifische Antikörper in einem komplexen Prozeß zusammen mit einer großen Anzahl weiterer Faktoren (Mediatoren) ausgeschüttet wie Interleukin 1 (IL 1), Interleukin 2 (IL 2), koloniestimulierender Faktor (CSF), migrationsinhibierender Faktor (MIF), Makrophagen aktivierender Faktor (MAF), Tumor nekrotisierender Faktor (TNF) Lymphotoxin (LT) und Fibroblastenwachstums-Faktor (FGF). Weitere Faktoren sind beschrieben von J.A. Georgiades et al. in Came, P.E. und Carter, W.A. (eds.), Interferons and Their Applications, Springer Verlag, 1984, und von Waksman, B.H. in Cohen, S. et al. (eds.), Biology of the Lymphokines, Academic Press Inc., 1979.

Diejenigen Faktoren, die von der Untergruppe der Lymphozyten gebildet werden, bezeichnet man allgemein als Lymphokine. Zu dieser Gruppe wird auch IFN-gamma gerechnet, das hauptsächlich von T-Lymphozyten produziert wird. Die Syntheseleistung der IFN-gamma produzierenden T-Lymphozyten wird ihrerseits wieder von dem Vorhandensein der oben erwähnten Faktoren beeinflußt.

Weiterhin existieren etablierte bzw. transformierte Zellinien, die IFN-gamma konstitutiv produzieren, wie von N. Fujii et al. in J. Immunology 130, 1983, S. 1683 - 1686, und A. Zlotnik et al. in J. Immunology 131, 1983, S. 794 - 800, beschrieben.

Ein bevorzugtes Verfahren zur Gewinnung von humanem Roh-IFN-gamma wird nachfolgend beschrieben. Als Ausgangsmaterial dienen lymphozytenreiche Plasmafraktionen von Blutkonserven, sogenannte "buffy coats", die gepoolt und durch schonende Zentrifugation bei 600 - 800 g von Plasmaresten befreit werden. Die pelletierten Gesamtleukozyten werden in vorgewärmtem Medium mit einer Dichte von 5 Millionen Zellen /ml suspendiert. Die Zellsuspension wird in aliquots in geeigneten Kultivierungsgefäßen nach Zugabe eines Mitogens (z. B. Phythämagglutinin) und Phorbolester [z. B. Phorbolmyristylacetat (PMA)] bis zu 70 Stunden bei 37°C auf einem Schüttler inkubiert. Die IFN-gamma-haltigen Kulturüberstände werden durch Zentrifugation gewonnen und können bis zur Weiterverwendung bei 4°C gelagert werden. Üblicherweise enthalten die so gewonnenen Präparationen etwa 10 000 Internationale Referenzeinheiten (I.E.) IFN-gamma pro ml.

Der IFN-gamma-Gehalt wird durch einen CPE-Reduktionstest in geeigneten Indikatorzellen (z. B. WISH) mit einem Testvirus (z. B. EMC der Maus) gegen den Referenzstandard Gg 23-901-530 des National Institute of Health (USA) als antivirale Aktivität des Präparates bestimmt.

Weitere Methoden zur Gewinnung von IFN-gamma-Präparationen werden von Y.K. Yip et al. in Infection and Immunity, 34, 1981, S. 131 - 139, und in den US-PS-4 376 821, 4 376 822 sowie 4 460 685 beschrieben.

Ein grundsätzlich anderer Weg zur Gewinnung von humanen IFN-gamma-Präparaten wird mit der Einschleusung des humanen Strukturgens mit Hilfe geeigneter Vektoren in heterologe Wirtszellen beschritten. Diese rekombinanten Gene werden konstitutiv oder nach Zugabe spezifischer Induktoren von der Wirtszelle exprimiert.

Auch wenn es sich bei dem Strukturgen um eine eindeutig definierte Sequenz aus Nukleotiden handelt, die zwingend zur Synthese einer daraus ableitbaren Aminosäuresequenz führt, ist das IFN-gamma-Molekül des mit Hilfe der Wirtszelle hergestellten Präparates nicht notwendigerweise mit einem natürlich vorkommenden IFN-gamma identisch. Diese Möglichkeit der Variation unter Erhaltung der spezifischen Wirkung durch "post processing" wird durch das sogenannte Proteindesign erweitert, durch das das Strukturgen durch chemische bzw. biochemische Vollsynthese oder Modifikation verändert werden kann.

Als Wirtszellen zur Aufnahme des Humangens können außer Bakterien (z. B. E. coli wie von G. Simons et al. in Gene 28, 1984 S. 55 - 64, beschrieben) auch Hefen (z. B. Saccharomyces cerevisiae, wie von R. Derynck et al. in Nucleic Acids Research 11, 1983 S. 1819 - 1837, beschrieben) oder eukaryotische Zellen (wie z. B. Chinese Hamster Ovary Cells, wie von S.J. Scahill et al. in Proc. Nat. Acad. Sci. USA 80, 1983 S. 4654 - 4658, Affenzellen, wie von P.W. Gray et al. in Nature 295, 1982 S. 503 - 508, beschrieben) dienen.

In einigen dieser Systeme reichert sich entweder IFN-gamma bis zu einer Konzentration von mehr als 100 000 I.E. pro ml in der Kulturflüssigkeit an oder es wird im Wirt selbst angereichert und stellt dort bis zu 25 Prozent des Proteingehalts der Zelle dar.

Die Anreicherung bzw. Reinigung der nach den vorstehend beschriebenen Verfahren erhaltenen IFN-gamma-Präparationen kann nach den folgenden Methoden einzeln oder in Kombination erfolgen:

1. Controlled Pore Glass (CPG) oder Silica-Gel;

2. Gelfiltration (z. B. AcA 54 oder Sephacel S 200);

3.
   Ionenaustauschchromatographie (CM-Sepharose oder Phosphocellulose oder DEAEcellulose);

4. Affinitätschromatographie (Con A-Sepharose oder Poly-U-Sepharose oder Cu-Chelat-Sepharose);

5. Immunaffinitätschromatographie (Anti-IFN-gamma-Sepharpse);

6. HPLC (z. B. mit Reversed Phase Materialien).

Entsprechende Verfahren wurden von Y.K. Yip et al, "Partial Purification and Characterization of Human Gamma (Immune) Interferon" in Proc. Nat. Acad. Sci. USA, 78, 1981 S. 1601 - 1605, oder von D. Novick et al., EMBO Journal 2, 1983 S. 1527 - 1530, oder in der DE-A-3 136 166 beschrieben.

Durch geeignete Kombinationen ist eine Reinigung bis zur elektrophoretischen Homogenität möglich; z. Zt. liegt die dabei erreichte maximale spezifische Aktivität bei 100 - 200 Millionen I.E., während die beschriebenen Durchschnittswerte bei 10 und 50 Millionen I.E. liegen.

Wie die anderen Interferone ist auch IFN-gamma eine Substanz mit antiviraler, antiproliferativer und immunmodulierender Wirkung. Aufgrund seiner in vitro stärkeren antiproliferativen Wirkung als IFN-alpha und IFN-beta wurde es bisher nahezu ausschließlich bei Patienten mit malignen Erkrankungen eingesetzt [Came, P.E. und Carter, W. A.: Interferons and their applications. Springer Verlag: Berlin, Heidelberg, New York, Tokyo, 1984; De Maeyer, E. D. und Schellekens, H. (Eds.): The biology of the interferon syste μm, Elsevier Science Publishers: Amsterdam, New York, Oxford, 1983].

Bei Rheuma handelt es sich um Schmerzen und Funktionseinschränkungen in den Bewegungsorganen (Mathies, H., Rheuma, Gustav Fischer Verlag: Stuttgart, New York 1983). Nach diagnostisch-therapeutisch-praktischen Gesichtspunkten werden unterschieden: entzündlicher Rheumatismus, degenerativer Rheumatismus und extraartikulärer Rheumatismus. Zum rheumatischen Formenkreis werden weiterhin gezählt: systemischer Lupus erythematodes, progressive systemische Sklerodermie, Dermatomyositis, Panarteriitis nodosa, Arthritis urica und Chondrokalzinose (Pschyrembel, W., Klinisches Wörterbuch, Walter de Gruyter: Berlin, New York 1982). Bei der zum entzündlichen Rheumatismus zählenden chronischen Arthritis wird die bei Kindern und Jugendlichen auftretende Form häufig von der der Erwachsenen unterschieden (Anzell, B.M., Juvenile chronic polyarthritis, Arthr. Rheum. Suppl. 20, 1977; 176 - 180, Truckenbrodt, H., Die juvenile chronische Arthritis und ihre Subgruppen, Münch. med. Wschr. 1984 126, 1076 - 1078.

Da die Ätiologie der weitaus meisten rheumatischen Erkrankungen nicht geklärt ist, steht eine kausale Therapie in der Regel nicht zur Verfügung (Krüger, K., Neue Therapieprinzipien in der Rheumatologie, Münch. med. Wschr. 1984 126, 1084 - 1086. In der medikamentösen Behandlung werden verschiedenartige Arzneimittel eingesetzt, die zwei Gruppen zugeordnet werden können: die symptomatischen Antirheumatika wie Salizylsäure, Indometacin und Glukokortikoide, die rasch, aber nur so lange wirken, wie die Mittel genommen werden, und die Basis-Antirheumatika wie Goldsalze, D-Penicillamin, Chloroquin und Immunsuppressiva, die in die Pathogenese eingreifen und erst einige Wochen nach Beginn der Therapie wirksam sind, deren Effekt aber auch noch nach Absetzen für einige Zeit anhält (Mathies, H. s.o.; Butler, R.C. und Goddard, D.H., Controversy in the treatment of rheumatoid arthritis, Lancet ii, 1984 278 - 279. Außerdem werden noch eine Reihe weiterer Substanzen in der Rheumatherapie eingesetzt wie z. B. die Thymushormone (Krüger, K., s.o.).

Eine mit IFN-alpha durchgeführte Therapie stellte sich als unwirksam gegen rheumatoide Arthritis heraus (Kajandar, A. et al., Interferon treatment of rheumatoid arthritis, Lancet i, 1979 984. Dieses Ergebnis ist nicht überraschend, da bei bestimmten rheumatischen Erkrankungen einschließlich der rheumatoiden Arthritis endogen Interferone gebildet werden, denen keine therapeutische Wirksamkeit zugesprochen wird oder die sogar für die Pathogenese dieser Erkrankungen mitverantwortlich gemacht werden. (Hooks, J.J. et al., Immune interferon in the circulation of patients with autoimmune disease. N. Engl. J. Med. 301, 1979; 5 - 8, Preble, O.T. et al., Systemic lupus erythematosus: presence in human serum of an unusual acid-labile leucocyte interferon, Science 216, 1982; 429 - 431, Degré, M. et al., Immune interferon in serum and synovial fluid in rheumatoid arthritis and releated disorders, Ann. Rheumatic Dis. 42, 1983; 672 - 676, Arvin, A.M. und Miller, J.J., Acid labile alphainterferon in sera and synovial fluids from patients with juvenile arthritis. Arthritis and Rheumatism 27, 1984; 582 - 585, Rosenbach T.O. et al., Interferon triggers experimental synovitis and may potentiate auto-immune disease in humans. Clin. Rheumatol. 3, 1984, 361 - 364.

Im Rahmen eigener klinischer Prüfungen konnte bei der Behandlung verschiedener rheumatischer Erkrankungen jedoch unerwartet gefunden werden, daß sowohl eine natürliche als auch eine rekombinante Interferon-gamma enthaltende Präparation nach wenigen Injektionen die Schmerzen anhaltend beseitigte und die Motorik der Patienten erheblich verbesserte. Interferon-gamma war therapeutisch wirksam bei entzündlichem Rheumatismus (Beispiel 1 - 3), extraartikulärem Rheumatismus (Beispiel 4 u. 5) und degenerativem Rheumatismus (Beispiel 6). Diese Ergebnisse überraschen, da die bisher klinisch gesicherten Wirkungen der Interferone dies nicht vermuten ließen und Interferon bei diesen Erkrankungen sogar als kontraindiziert angesehen wurde.

Natürliches IFN-gamma und rekombinantes IFN-gamma enthaltende Präparationen können wie folgt appliziert werden: intravenös als Bolus oder bis zu einer 24-stündigen Dauerinfusion, intramuskulär (einschließlich paravertebral bzw. periartikulär), intrasynovial, intraartikulär, periostal, subkutan, intrakutan, intrathekal, oral oder topisch, z. B. in einer Salben- oder Gelgrundlage durch Auftragen auf oder Einmassieren in die Haut. Bei diesen Applikationsformen können auch die dem Fachmann bekannten Depotformen verwendet werden.

Bei allen Applikationen werden je nach Schwere der Erkrankung gegeben: 200 Internationale Einheiten (I.E.) bis 2 000 Millionen I.E. ein oder mehrmalig an einem Tag oder an mehreren Tagen. Bei mehrtägiger Gabe können ein oder mehrere Dosen

a) an aufeinanderfolgenden Tagen
b) alle 2 bis 6 Tage
c) einmal pro Woche
d) alle zwei bis vier Wochen
e) einmal pro Monat oder
f) jedesmal bei Auftreten von Schmerzen

verabreicht werden.

Für die praktische Anwendung empfiehlt es sich, die Behandlung mit niedrigen Dosen zu beginnen, beispielsweise mit Dosen von 20 000 bis 2 000 000 I.E. bzw. ca. 2 µg bis 200 µg IFN-gamma.

Zur Steigerung der Effektivität der Interferon-gamma-Präparation können folgende Substanzen zusätzlich in Frage:

a) andere Interferone und/oder andere von Leukozyten gebildete bzw. durch gentechnologische Verfahren hergestellte Zell-Mediatoren wie IL 1, IL 2, CSF, MIF, MAF, TNF, LT und FGF.
b) in der Therapie bisher verwendete Antirheumatika einschließlich der Thymushormone.

Zur Herstellung der jeweiligen Darreichungsform werden die dem Fachmann bekannten Hilfsstoffe verwendet.

**Beispiel 1**

Patient:
Lu. männl.
Diagnose:
schwere chronische Polyarthritis seit über 10 Jahren, Deformation an Händen und Füßen; bei Einlieferung in die Klinik nicht bewegungsfähig; bisher keine Besserung des Leidens möglich.
Substanz:
humane IFN-gamma-Präparation aus menschlichen Leukozyten
Applikationsweise:
subkutan

Therapieschema:

| Behandlungstag | Dosierung |
|---|---|
| 1. | $0,1 \times 10^6$ I.E. |
| 2. | $0,5 \times 10^6$ I.E. |
| 3. | $0,5 \times 10^6$ I.E. |
| 4. | $1,0 \times 10^6$ I.E. |
| 5. | $1,0 \times 10^6$ I.E. |
| 8. | $1,5 \times 10^6$ I.E. |
| 9. | $1,5 \times 10^6$ I.E. |
| 10. | $1,5 \times 10^6$ I.E. |
| 11. | $1,5 \times 10^6$ I.E. |

Ergebnis:
Am 6. Behandlungstag zeigte sich eine wesentliche Besserung der Schmerzen in den unteren Extremitäten; ab dem 11. Behandlungstag war der Patient völlig schmerzfrei. Er war gehfähig und konnte sogar Treppen steigen. Die Klopfempfindlichkeit war verschwunden.

**Beispiel 2**

Patient:
Zi. männl.
Diagnose:
schwere chronische Polyarthritis mit Deformation an beiden Händen
Substanz:
humane IFN-gamma-Präparation aus menschlichen Leukozyten

Applikationsweise:s
subkutan

Therapieschema:

| Behandlungstag | Dosierung |
|---|---|
| 1. | $0,5 \times 10^6$ I.E. |
| 2. | $0,5 \times 10^6$ I.E. |
| 3. | $0,5 \times 10^6$ I.E. |
| 4. | $0,5 \times 10^6$ I.E. |
| 5. | $0,5 \times 10^6$ I.E. |
| 8. | $1,0 \times 10^6$ I.E. |
| 9. | $1,0 \times 10^6$ I.E. |
| 10. | $1,0 \times 10^6$ I.E. |

Ergebnis:
Unter der Therapie mit $0,5 \times 10^6$ I.E. trat eine zunehmende Besserung der Schmerzen ein, unter der Therapie mit $1,0 \times 10^6$ I.E. wurde der Patient dann völlig schmerzfrei. Der Patient konnte zu Beginn der Therapie maximal 4 Minuten auf einem Heimtrainer radfahren, ab dem 9. Behandlungstag 2 x 20 Minuten. Der Patient fühlte sich subjektiv wohl und benötigte kein Cortison mehr. Er wurde am 17. Tag nach Behandlungsbeginn aus der Klinik entlassen. Der Patient war am Entlassungstag schmerzfrei, seine Beweglichkeit einwandfrei; über den Gelenken existierte keine Klopfempfindlichkeit mehr.

**Beispiel 3**

Patient:
I. Jä. weibl.
Diagnose:
seronegative rheumatische Polyarthritis, Anämie, lokal nodale Hyperplasie der Leber
Substanz:
humane IFN-gamma-Präparation aus E. coli
Applikationsweise:
intramuskulär

Therapieschema:

| Behandlungstag | Dosierung |
|---|---|
| 1. | 100 µg Wirkstoff |
| 2. | 100 µg Wirkstoff |
| 3. | 100 µg Wirkstoff |
| 4. | 100 µg Wirkstoff |
| 5. | 100 µg Wirkstoff |
| 6. | 100 µg Wirkstoff |
| 7. | 100 µg Wirkstoff |
| 8. | 100 µg Wirkstoff |
| 11. | 175 µg Wirkstoff |
| 12. | 175 µg Wirkstoff |
| 13. | 50 µg Wirkstoff |
| 14. | 50 µg Wirkstoff |

Ergebnis:
Nachdem sich in den ersten Therapietagen die Schmerzen verstärkten, war die Patientin am 4. Behandlungstag erstmals schmerzfrei. Im weiteren Verlauf der Therapie traten die Schmerzen nur noch kurzfristig auf. Seit kurzem erhält die Patientin einmal pro Woche 10 µg Wirkstoff i.m. Die Patientin verträgt das Medikament gut und ist seither wieder schmerzfrei.

**Beispiel 4**

Patient:
H. L. weibl.
Diagnose:
gesicherte rheumatoide Arthritis seit 4 Jahren. Betroffen sind Hände, Hüften, Knie, Sprunggelenke und Zehengrundgelenke. Patientin refraktär gegen Gold und Prednisolon.
Substanz:
humane IFN-gamma-Präparation aus E. coli
Applikation:
subcutan
Dosis:
2 mal 250 µg täglich als Initialtherapie; 2 mal 50 µg täglich als Erhaltungstherapie.
Ergebnis:
Nach 5 Tagen Behandlung war die Patientin schmerzfrei und frei beweglich. Unter der Erhaltungstherapie besserte sich das klinische Bild der rheumatoiden Arthritis weitgehend und anhaltend. Die Patientin war schmerzfrei.

**Beispiel 5**

Patient:
W.D. weibl.
Diagnose:
Periarthritis humeroscapularis seit 1 Woche
Substanz:
humane IFN-gamma-Präparation aus menschlichen Leukozyten
Applikationsweise und Therapieschema:
einmalig subkutan (0,5 x $10^6$ I.E.)
Ergebnis:
Die Patientin war innerhalb weniger Minuten schmerzfrei. Seither sind keine Schmerzen mehr aufgetreten.

**Beispiel 6**

Patient:
L. L. männl.
Diagnose:
Ischialgie $L_5/S_1$ rechts; Osteoporose seit 3 Wochen
Substanz:
humane IFN-gamma-Präparation aus menschlichen Leukozyten
Applikationsweise:
paravertebral

Therapieschema:

| Behandlungstag | Dosierung |
|---|---|
| 1. | 0,5 x $10^6$ I.E. |
| 21. | 0,5 x $10^6$ I.E. |

Ergebnis:
Nach der ersten Injektion war der Patient 5 Tage lang, nach der zweiten Injektion bis heute (9 Monate nach erstem Behandlungstag) schmerzfrei.

**Patentansprüche**

1. Verwendung von Interferongamma zur Herstellung von Präparationen zur Behandlung rheumatischer Erkrankungen.

2. Verwendung von Interferongamma nach Anspruch 1 zur Herstellung von Präparationen zur Behandlung von entzündlichem Rheumatismus.

3. Verwendung von Interferongamma nach Anspruch 2 zur Herstellung von Präparationen zur Behandlung von chronischer Polyarthritis (rheumatoider Arthritis).

4. Verwendung von Interferongamma nach Anspruch 2 zur Herstellung von Präparationen zur Behandlung von juveniler chronischer Arthritis.

5. Verwendung von Interferongamma nach Anspruch 2 zur Herstellung von Präparationen zur Behandlung der Psoriasis arthropathica.

6. Verwendung von Interferongamma nach Anspruch 1 zur Herstellung von Präparationen zur Behandlung von extraartikulärem Rheumatismus (Weichteilrheumatismus).

7. Verwendung von Interferongamma nach Anspruch 6 zur Herstellung von Präparationen zur Behandlung von Muskelrheumatismus und Periarthritis humeroscapularis.

8. Verwendung von Interferongamma nach Anspruch 1 zur Herstellung von Präparationen zur Behandlung von degenerativem Rheumatismus.

9. Verwendung von Interferongamma nach Anspruch 1 zur Herstellung von Präparationen zur Behandlung von Lupus erythematodes.

10. Verwendung von Interferongamma nach Anspruch 1 zur Herstellung von Präparationen zur Behandlung von Dermatomyositis.

11. Verwendung von Interferongamma nach Anspruch 1 zur Herstellung von Präparationen zur Behandlung von Sklerodermie.

12. Verwendung von Interferongamma nach einem der Ansprüche 1 bis 11, dadurch gekennzeichnet, daß das Interferongamma von menschlichen Zellen oder durch gentechnologische Verfahren erhalten wurde.

13. Verwendung von Interferongamma nach einem der Ansprüche 1 bis 11 zur Herstellung von Präparationen zur Behandlung rheumatischer Erkrankungen, wobei diese Präparationen zusätzlich andere Interferone und/oder andere durch Leukozyten gebildete oder durch gentechnologische Verfahren hergestellte Zell-Mediatoren enthalten.

14. Verwendung von Interferongamma nach einem der Ansprüche 1 bis 13 zur Herstellung von Präparationen in einer intravenös, intramuskulär, intraartikulär, intrasynovial, periostal, subkutan, intrakutan, intrathekal, oral oder topisch zu verabreichenden Applikationsform.

15. Verwendung von Interferongamma zur Herstellung von Präparationen nach Anspruch 14 in einer für Polypeptidtherapeutika üblichen Depotform.

16. Verwendung von Interferongamma zur Herstellung von Präparationen nach einem der Ansprüche 1 bis 15, dadurch gekennzeichnet, daß die verabreichungsfertige Einzeldosis 200 Internationale Referenzeinheiten (I.E.) bis 2000 Millionen I.E. (etwa 20 ng bis 20 mg) Interferongamma enthält.

17. Verwendung von Interferongamma zur Herstellung von Präparationen nach einem der Ansprüche 1 bis 16, dadurch gekennzeichnet, daß diese Präparationen zusätzlich symptomatisch wirkende Antirheumatika, Basisantirheumatika und/oder Thymus-Hormone enthalten.

**Revendications**

1. Utilisation de l'interféron gamma pour la préparation de compositions pour le traitement des affections rhumatismales.

2. Utilisation de l'interféron gamma selon la revendication 1 pour la préparation de compositions pour le traitement du rhumatisme inflammatoire.

3. Utilisation de l'interféron gamma selon la revendication 2 pour la préparation de compositions pour le traitement de la polyarthrite chronique (arthrite rhumatoïde).

4. Utilisation de l'interféron gamma selon la revendication 2 pour la préparation de compositions pour le traitement de l'arthrite chronique juvénile.

5. Utilisation de l'interféron gamma selon la revendication 2 pour la préparation de compositions pour le traitement du psoriasis arthropathique.

6. Utilisation de l'interféron gamma selon la revendication 1 pour la préparation de compositions pour le traitement du rhumatisme articulaire.

7. Utilisation de l'interféron gamma selon la revendication 6 pour la préparation de compositions pour le traitement du rhumatisme musculaire et de la périarthrite huméroscapulaire.

8. Utilisation de l'interféron gamma selon la revendication 1 pour la préparation de compositions pour le traitement du rhumatisme dégénératif.

9. Utilisation de l'interféron gamma selon la revendication 1 pour la préparation de compositions pour le traitement du lupus erythémateux.

10. Utilisation de l'interféron gamma selon la revendication 1 pour la préparation de compositions pour le traitement de la dermatomyosite.

11. Utilisation de l'interféron gamma selon la revendication 1 pour la préparation de compositions pour le traitement de la sclerodermie.

12. Utilisation de l'interféron gamma selon l'une des revendications 1 à 11, caractérisée en ce que l'interféron gamma a été obtenu à partir de cellules humaines ou par un procédé de technologie ou de génie génétique.

13. Utilisation de l'interféron gamma selon l'une des revendications 1 à 11 pour la préparation de compositions pour le traitement de maladies rhumatismales, où ces compositions contiennent encore d'autres interférons et/ou d'autres médiateurs cellulaires formés par des leucocytes ou obtenus par des procédés de technologie ou de génie génétique.

14. Utilisation de l'interféron gamma selon l'une des revendications 1 à 13 pour la préparation de compositions sous une forme convenant pour l'application par la voie intraveineuse, intramusculaire, intraarticulaire, intrasynoviale, périostée, sous-cutanée, intracutanée, intrathécale, orale ou topique.

15. Utilisation de l'interféron gamma pour la préparation de compositions selon la revendication 14 sous une forme de dépôt usuelle en thérapeutique polypeptidique.

16. Utilisation de l'interféron gamma pour la préparation de compositions selon l'une des revendications 1 à 15, caractérisée en ce que la dose individuelle prête à l'administration contient de 200 unités internationales de référence (UI) à 2000 millions de UI (20 ng à 20 mg) d'interféron gamma.

17. Utilisation de l'interféron gamma pour la préparation de compositions selon l'une des revendications 1 à 16 caractérisée en ce que ces préparations contiennent en outre des hormones de thymus, des agents antirhumatismaux de base et/ou des agents antirhumatismaux à action symptomatique.

## Claims

1. Use of gamma interferon in the manufacture of preparations for the treatment of rheumatic diseases.

2. Use of gamma interferon according to Claim 1, in the manufacture of preparations for the treatment of inflammatory rheumatism.

3. Use of gamma interferon according to Claim 2, in the manufacture of preparations for the treatment of chronic polyarthritis (rheumatoid arthritis).

4. Use of gamma interferon according to Claim 2, in the manufacture of preparations for the treatment of juvenile chronic arthritis.

5. Use of gamma interferon according to Claim 2, in the manufacture of preparations for the treatment of psoriasis arthropathica.

6. Use of gamma interferon according to Claim 1, in the manufacture of preparations for the treatment of extraarticular rheumatism (soft part rheumatism).

7. Use of gamma interferon according to Claim 6, in the manufacture of preparations for the treatment of muscular rheumatism and periarthritis humeroscapularis.

8. Use of gamma interferon according to Claim 1, in the manufacture of preparations for the treatment of degenerative rheumatism.

9. Use of gamma interferon according to Claim 1, in the manufacture of preparations for the treatment of lupus erythematodes.

10. Use of gamma interferon according to Claim 1, in the manufacture of preparations for the treatment of dermatomyositis.

11. Use of gamma interferon according to Claim 1, in the manufacture of preparations for the treatment of sclerodermia.

12. Use of gamma interferon according to any one of Claims 1 to 11, characterised in that the gamma interferon is obtained from human cells or by genetic engineering methods.

13. Use of gamma interferon according to any one of Claims 1 to 11, in the manufacture of preparations for the treatment of rheumatic diseases, wherein these preparations additionally contain interferons and/or other cell mediators formed by leucocytes or produced by genetic engineering methods.

14. Use of gamma interferon according to any one of Claims 1 to 13, in the manufacture of preparations in a form of application to be administered intravenously, intramuscularly, intraarticularly, intrasynovially, periostally, subcutaneously, intracutaneously, intrathecally, orally or topically.

15. Use of gamma interferon in the manufacture of preparations according to Claim 14, in a form of depot conventional for polypeptide therapeutic agents.

16. Use of gamma interferon in the manufacture of preparations according to any one of Claims 1 to 15, characterised in that an individual dose ready to be administered contains 200 international reference units (I. E.) to 2000 million I. E. (20 ng to 20 mg) of gamma interferon.

17. Use of gamma interferon in the manufacture of preparations according to any one of Claims 1 to 16, characterised in that these preparations additionally contain symptomatically acting antirheumatic agents, basal antirheumatic agents and/or thymus hormones.